# EUROPEAN PATENT SPECIFICATION

(11) **EP 4 230 167 B1**
(45) Date of publication and mention of the grant of the patent: **11.09.2024**
(21) Application number: 23158078.8
(22) Date of filing: 22.02.2023
(51) Int. Cl.: A61B 18/20

(54) **DEVICE FOR LASER TATTOO OR SCAR REMOVAL AND RESPECTIVE ONCOLOGICAL APPLICATIONS**
VORRICHTUNG ZUR LASERENTFERNUNG VON TÄTOWIERUNG ODER NARBEN UND ENTSPRECHENDE ONKOLOGISCHE ANWENDUNGEN
DISPOSITIF POUR L'ÉLIMINATION DU TATOUAGE OU DE CICATRICES LASER ET APPLICATIONS ONCOLOGIQUES RESPECTIVES

(30) Priority: 22.02.2022 PT 2022117806
(43) Date of publication of application: 23.08.2023
(73) Proprietor: IPCA - Instituto Politécnico do Cávado e do Ave, 4750-810 V Frescainha (S Martinho) (PT)
(72) Inventor: ARAÚJO MARTINS VILAÇA, João Luís, 4750-810 V Frescainha (S Martinho) (PT); GOMES OLIVEIRA, Bruno Miguel, 4750-810 V Frescainha (S Martinho) (PT); GONÇALVES MORAIS, Pedro André, 4750-810 V Frescainha (S Martinho) (PT)
(74) Representative: Patentree

(56) References cited:
- WO-A1-2021/059255

## Description

### TECHNICAL FIELD

The present disclosure relates to a laser device for laser tattoo or scar removal treatment.

### BACKGROUND

Tattooing is a method of incorporating colored inks into the dermis layer of skin. This method permanently colors the skin. The method requires a controlled application of the inks into the dermis layer by repeatedly perforating the epidermis layer with controlled punctures by needles coated in ink and the cells are stained with the desired pigments.

Recently, there has been a huge interest in developing an effective, safety and noninvasive strategy for tattoo removal. In this scope, laser therapy has been implemented and is now widely. In this therapy, very short and strong pulses penetrate into the skin, absorbed by the pigments in the ink. As the laser light is transformed into photoacoustic waves, it mechanically breaks up the ink into smaller particles. Then, the small particles are naturally absorbed by the body's own immune system. Specifically, for tattoo removal, top results can now be achieved using laser. In these, the ability to selectively target specific structures is made possible by adjusting several laser parameters, including its wavelength, spot size, cooling, pulse time, and power. The type of laser used generally depends upon the pigment colors.

Different lasers or different settings of the same laser are needed for different colors. The laser selectively targets and removes the tattoo without damaging the surrounding tissue, which greatly decreases scarring. Most tattoos do require multiple treatments. Black tattoo pigment absorbs all laser wavelengths, making it the easiest color to treat. Other colors can only be treated by selected lasers based upon the pigment color.

Several clinical studies have been made to understand how these parameters should be adjusted to achieve the best performance for laser-based tattoo removal. These adjustments depend on the correct identification and segmentation of the tattoo region (e.g. size, colour), as well as on the correct handling of the laser (e.g. distance/angle, vesselcentred). However, in clinical practice, naked-eye evaluation is still widely used to select the optimal parameters and to handle the device, being the treatment outcome highly dependent on the physician's expertise. Indeed, an incorrect use of the laser device reduces the treatment effectiveness and is the cause of several side effects, including discolouration, hypopigmentation, skin texture changes, and scarring.

WO2012135828 discloses a method and apparatus for treatment of health and skin conditions through the use of combination of laser systems capable of delivering different pulse durations.

WO200889344 discloses a device comprising an energy source capable of generating short bursts of energy at a variable pulse repetition rate. The device produces patterns of micro-disruptions in the skin. Furthermore, WO2021/059255 describes an image-guided robotic system for laser removal of tattoos.

During the last years, several research teams focused on the development or improvement of the laser technology itself and not in the improvement of the handling of the system, e.g. through collaborative solutions. Overall, a system that guarantees or markedly improves the correct handling of the laser during the entire treatment, minimizes the side effects of this treatment, improve its effectiveness and reduces the operator learning curvature, having the potential to be widely well accepted in a large number of laser/light-base treatments, representing a market share of more than 12 billion dollars in sales worldwide, is the aim of the present disclosure.

These facts are disclosed in order to illustrate the technical problem addressed by the present disclosure.

### GENERAL DESCRIPTION

The invention is defined by a device for laser removal of a tattoo or a scar extending along a pre-defined skin area as defined in claim 1.

This disclosure includes a method and device for guidance or automatic laser-based treatments for tattoo removal (i.e. removal of pigments stained in skin cells) or scars removal preferably for oncological patients who have scars from surgeries such as breast surgery. The scarring of the breast can be of some concern to woman post breast surgery and the lasers can be used to reduce the visibility of the scars. Optionally, the device and method can be used for applying or removing pigments for skin reconstruction for oncological patients, preferably for the applying or removal of pigmented skin that form the areola of a breast. The areola is the pigmented area on the breast around the nipple, which can range from pink to red to brown to dark brown or nearly black. Breast reconstruction is a surgery to recreate breasts after mastectomy. In some cases, it is needed to reshape the opposed breast (i.e. the healthy breast) or the implant, including the areola of the nipple, to guarantee a good symmetry between the implant and the opposed breast.

Such tool provides an automatic identification of tattoos or scars and a collaborative robotic handling during the treatment session. Thus, with the introduction of this technology, a minimization of side-effects, improvement of treatment effectiveness, and an optimization of treatment time through the reduction of time is obtained. Moreover, a reduction on the learning curvature is also obtained, broadening the procedure to novice users.

This disclosure includes a device and method that combine real-time segmentation of tattoos or scars with a collaborative robot for laser treatment. The proposed strategy automatically defines the optimal trajectories, shoot positions, and treatment stages (i.e. per tattoo colour, scar) in order to minimize treatment time and overshooting.

Then, it helps the physician to maintain an optimal laser handling throughout the entire treatment session. Thus, the main goals/contributions of this disclosure include:
Development of a strategy for tattoo or scar identification and segmentation from image acquisition systems (e.g. RGB, near-infrared camera - NIR with multispectral light/filters);
Design and development of a user-friendly augmented reality interface for real-time guidance of the laser treatment;
Development of a method to recognize the limits of the tattoo or scar to treatment, consequently minimizing side effects related with over-treatment;
Development of local control strategies for collaborative robotic laser guidance based on the location of the tattoo or scar to treat (laser/skin distance and angle, laser/tattoo positioning), to improve the handling precision and accuracy of the laser;
Development of a handheld gripper device to interact with the collaborative robot, which may include RGB/NIR/3D sensors and the laser device.

Furthermore, it is disclosed a device for laser removal of a tattoo or scar extending along a pre-defined skin area, comprising:
a skin treatment multi-laser exchangeable head having at least one laser beam target line, and a robotic support for supporting said laser head;
further comprising, attached to the exchangeable multi-laser head or to the robotic support where the exchangeable multi-laser head is supported:
   a multispectral camera for capturing skin surface images having a plurality of ranges of colour wavelength of the skin surface image, each range corresponding to a laser of the exchangeable multi-laser head;
   a mechanism configured to automatically exchange the laser head for changing between laser beams of the exchangeable multi-laser head;
   a robotic arm with fiber optics, in particular integrated;
   one or more handles for handling by the operator;
   a force sensor for sensing the intensity and direction of force applied to the handle or handles by the operator for controlling the robotic support, and
   an electronic data processor configured for carrying out the steps comprising:
      capturing an image of skin surface comprising the tattoo to be removed, by the multispectral camera;
      segmenting the captured image according to the plurality of ranges of colour wavelength by dividing said capture image according to each range of colour wavelength;
      defining a path from each said segmented captured image, said path comprising a plurality of path segments wherein each path segment corresponds to a colour wavelength range;
      selecting each path segment from the plurality of defined path segments;
      selecting the laser of the exchangeable multi-laser head according to the colour wavelength corresponding to each selected path segment;
      removing the tattoo or scar by receiving a user trigger input for shooting the exchangeable multi-laser head into a laser spot regularly spaced along each selected path segment.

In an embodiment, said path further comprises a plurality of connecting curve segments, each curve between two segmented paths wherein the curve connecting path has been obtained by using the maximum speed and acceleration of the robot arm, in particular the maximum speed and acceleration for safe operation, as defined to have a safe treatment.

In an embodiment, the electronic data processor is configured for carrying out the step of applying, through the robotic support, a transversal force, to the exchangeable multi-laser head along a transversal direction to said path and towards said selected path while allowing freely controllable movement of the robotic support along the direction of said selected path.

In an embodiment, the electronic data processor is configured for carrying out the step of applying, automatically by the robot, a transversal direction to the path and towards said path of the exchangeable multi-laser head.

In an embodiment, the exchangeable multi-laser head is linear or rotatable. Preferably the exchangeable multi-laser head is a rotatable multi-laser head that allows the axis to be always the same.

In an embodiment, said multispectral camera comprises a plurality of wavelengths light sources and filters to highlight different tattoo pigment colour or scar.

In an embodiment, said laser head comprises one or more lasers and/or one or more wavelengths.

In an embodiment, said optimal trajectories, are defined by the detected tattoo or scar and respective colours. The colours are grouped by its respective wavelength (closest to that specific colour in the electromagnetic spectrum). Then, the beam shape and size, the necessity to avoid overshooting and the tattoo area to be covered are used as cost variables of an optimization process to find the shortest path. Said shortest path method, should define the path (i.e., defined by control laser shoot points) that minimizes overshooting, number of shoots, and untreated total area of the tattoo.

In an embodiment, said shortest path method should pass on all the control points defined on the patient skin. Such control points must be spaced by a distance equal or higher to the spot size (i.e., laser parameter). Then, the trajectory is defined as the smallest path that avoids discontinuity problems on the velocity profile of the robot, having a parabolic profile when the distance between the next control point is less than a predefined distance amount. Moreover, said predefined distance amount is given by the maximum acceleration and velocity defined to have a safe treatment.

In an embodiment, said trajectory computation should avoid the robot movement to stop between consequent points, maintaining a smooth path and velocities, reducing the power consumption, and guaranteeing the velocities and accelerations to be within the limits of safeness.

In an embodiment, said treatment trajectory is continuously updated to remove the influence of patient and robot movement by fusing a real-time image tracking method with the rigid transformation related to the robot movement (i.e. modification of the robot position/orientation in each axis). According to the robot current position and the next control shoot point to be addressed, new trajectories are computed having into account the limits of acceleration and velocity pre-defined to have a safe treatment. Said trajectories path must always guarantee that the robot pass in all the control shoot points on the skin.

In an embodiment, said treatment trajectories are computed using previous treatment prior information (e.g., acquisition of the target tattoo obtained from previous laser treatments), guaranteeing that the relative position between the laser device and the entire treatment region is always known, wherein said treatment region can be partial occluded or not completely seen during the treatment by the image sensors.

In an embodiment, said control points of the treatment trajectories are computed having into consideration control points from previous treatments, wherein said control points of the treatment trajectories are defined to maximize the tattoo area treated between consecutive treatments.

In an embodiment, said transversal direction is parallel to the skin surface.

In an embodiment, the electronic data processor is further configured for maintaining, through the robotic support, a first transversal distance between said path and where the laser beam target line intersects the skin surface, wherein said first transversal distance varies positively with a first force as measured by said force sensor along said transversal direction parallel to the skin surface.

In an embodiment, said first transversal distance is directly proportional to said force.

In an embodiment, the electronic data processor is further configured for applying, through the robotic support, said transversal force, to the laser head along a transversal direction to said path and towards said path, to maintain, constant and equal to a predetermined amount, the first transversal distance between said path and where the laser beam target line intersects the skin surface.

In an embodiment, the electronic data processor is further configured for maintaining, through the robotic support, a second transversal distance between said laser head and skin surface, along a further transversal direction, wherein said further transversal direction is perpendicular to the skin surface, and wherein said second transversal distance, offset by a predetermined amount of distance, varies positively with a second force as measured by said force sensor along said further transversal direction perpendicular to the skin surface.

In an embodiment, said second transversal distance is directly proportional to said further force.

In an embodiment, the proportionality constant of the first transversal distance relative to the first force and the proportionality constant of the second transversal distance relative to the second force are different, in particular the proportionality constant of the first transversal distance relative to the first force is higher than the proportionality constant of the second transversal distance relative to the second force.

In an embodiment, the electronic data processor is further configured for applying, through the robotic support, a further force to said laser head along a further transversal direction to said path and towards said path, wherein said further transversal direction is perpendicular to the skin surface, such that a second transversal distance between laser head and skin surface is kept constant and equal to a predetermined amount.

In an embodiment the electronic data processor is further configured for applying a speed dampening to the movement of the robotic support which is inversely related to the speed of the multi-laser exchangeable head.

In an embodiment, the transversal force is determined by an impedance control in respect of the transversal distance, parallel to the skin surface, between said path and where the laser beam target line intersects the skin surface, versus the force applied by the operator in said transversal direction parallel to the skin surface.

In an embodiment, the transversal force is determined by an impedance control in respect of the transversal distance, perpendicular to the skin surface, between said laser head and skin surface, offset by a predetermined amount of distance, versus the force applied by the operator in said transversal direction perpendicular to the skin surface.

In an embodiment, the electronic data processor is further configured for applying, through the robotic support, a further force to said laser head along a further transversal direction to said path and where the laser beam target line intersects the skin surface, such that a second transversal distance between laser head and the laser beam target line is kept constant and equal to a predetermined amount.

In an embodiment, the electronic data processor is further configured for maintaining, through the robotic support, a first angular displacement of the laser beam head about the longitudinal direction to said path, wherein said angular displacement varies positively with a first torque as measured by said force sensor along said longitudinal direction to the said path.

In an embodiment, the electronic data processor is further configured for maintaining, through the robotic support, a second angular displacement between laser beam head and a transversal direction to said path which is perpendicular to the skin surface, wherein said angular displacement varies positively with a second torque as measured by said force sensor along said transversal direction.

In an embodiment, the electronic data processor is further configured for maintaining, through the robotic support, a third angular displacement between laser beam head and a transversal direction to said path which is parallel to the skin surface, wherein said angular displacement varies positively with a third torque as measured by said force sensor along said transversal direction.

In an embodiment, said torque is determined by an impedance control in respect of the angular displacement between the laser beam head and the longitudinal direction to the said path, versus the torque applied by the operator in said angular displacement.

In an embodiment, said torque is determined by an impedance control in respect of the angular displacement between the laser beam head and a transversal direction to said path which is perpendicular to the skin surface, versus the torque applied by the operator in said angular displacement.

In an embodiment, said torque is determined by an impedance control in respect of the angular displacement between the laser beam head and a transversal direction to said path which is parallel to the skin surface, versus the torque applied by the operator in said angular displacement.

In an embodiment, the electronic data processor is further configured for applying, through the robotic support, a longitudinal force to said laser head along a longitudinal direction along said path, wherein said force is determined by an impedance control in respect of the longitudinal distance along said path from a predetermined treatment point in respect of the force applied by the operator in said longitudinal direction, wherein the transversal movement stiffness is higher than the longitudinal movement stiffness.

In an embodiment, there are a plurality of predetermined treatment points along said path, and the distance between predetermined treatment points is kept constant and equal to a predetermined amount.

In an embodiment, the electronic data processor is further configured to automatically change between control points after the current control point is treated.

In an embodiment, the electronic data processor is further configured for applying through the robotic support a torque to said laser head, such that the angle between the beam of the laser head and skin surface is kept perpendicular.

In an embodiment, the electronic data processor is further configured for applying, through the robotic support, a force to said laser head longitudinally to said path such that the multi-laser exchangeable head is freely movable along said path by the operator using said handle or handles.

In an embodiment, the electronic data processor is further configured for applying, through the robotic support, a force to said laser head longitudinally to said path such that the distance between the laser head and the current treatment point is kept constant and equal to a predetermined amount.

In an embodiment, said longitudinal force is determined by an impedance control in respect of the distance between laser beam head and the current treatment point, versus the force applied by the operator in said direction.

In an embodiment, the electronic data processor is further configured for applying, through the robotic support, a force to said laser head along a direction between the point where the laser beam target line intersects the skin surface and the current treatment point wherein the movement stiffness is defined to apply a smooth movement towards the said current treatment point.

In an embodiment, the electronic data processor is further configured to shoot the laser when the distance between the current position and the treatment point is equal or less to a predefined constant, wherein this distance is defined to guarantee the safeness of the treatment.

In an embodiment, the electronic data processor is further configured to automatically change between treatment points after the current treatment point be treated (i.e. after a laser shoot).

In an embodiment, the robotic support is a robotic arm, in particular a robotic arm comprising 6 or 7 degrees of freedom.

In an embodiment the device comprises a sloped base for holding the robotic support, wherein the sloped base is inclined towards the treatment area.

In an embodiment, the electronic data processor is further configured for using low tolerance configurations of the robotic arm degrees of freedom.

In an embodiment comprises a multispectral camera for tattoo or scar image capture enhancement. A multispectral camera can capture and/or enhance tattoo or scar images up to a few millimetres inside the skin. Moreover, it highlights different tattoo or scar colours, improving its detection by using different light filters and light sources (e.g. around 532, 1064, 585, 650 nm). This is advantageous for the present disclosure because there is an improved tattoo visualization.

In an embodiment, the device comprises a VISible light, VIS, camera for skin surface image capture.

In an embodiment, the force sensor is mounted to sense intensity and direction of the force applied by the operator directly to the handle or handles, or mounted to sense intensity and direction of the force applied by the operator indirectly to the handle or handles by sensing the intensity and direction of force applied by the operator to a joystick mounted on the handle or handles. The force sensor can sense the intensity and direction of force applied to the handle or handles by the operator, either directly on the handle or handles, or indirectly by sensing the intensity and direction of force applied by the operator to a joystick mounted on the handle or handles.

In an embodiment, the device comprises a force sensor that measures the force applied by the user directly in the robotic support of said multi-laser head and/or in a joystick.

In an embodiment, the electronic data processor is further configured for combining the NIR and VIS captured images.

In an embodiment, the device comprises a sstereoscopic camera, a LIDAR camera or a structured light camera for 3D capture of the skin surface.

In an embodiment, the device comprises 3 or more laser rangefinders arranged at, or in the vicinity of, the laser head for determining the distance between the laser head and the skin surface, or for determining the angle between the laser head beam and the skin surface or their combination.

In an embodiment, the device comprises 3 or more laser rangefinders arranged at, or in the vicinity of, the multi-laser head in order to overlay the rangefinder laser spots in the captured skin surface image for determining pixel size of the skin surface image and/or for aligning the captured skin surface image with the multi-laser exchangeable head or for their combination.

In an embodiment, an electronic data processor is further configured for applying through the robotic support a force to said laser head along a transversal direction to said path and towards said path, wherein said transversal direction is perpendicular to the skin surface, such that the distance between laser head and skin surface is kept constant and equal to a predetermined amount minus a predetermined tattoo depth.

In an embodiment, the robotic support is automatically moved or is operated by collaborative motion between an operator and said robotic support.

It is further disclosed a method for laser removal of a tattoo extending along a predefined skin, by operating a device comprising: a skin treatment multi-laser exchangeable head having at least one laser beam target line, and a robotic support for supporting said laser head; further comprising, attached to the laser head or to the robotic support where the laser head is supported: a multispectral camera for capturing skin surface images having plurality of ranges of colour wavelength of the skin surface image, each range corresponding to a laser of the exchangeable multi-laser head, a mechanism configured to automatically exchange the laser head for changing between laser beams of the multi-laser exchangeable head, a robotic arm comprising fiber optics for laser treatment integrated; one or more handles for handling by the operator, a force sensor for sensing the intensity and direction of force applied to the handle or handles by the operator for controlling the robotic support and an electronic data processor;
the method comprising the electronic data processor carrying out the steps of:
capturing an image of skin surface comprising the tattoo to be removed, by the multispectral camera;
segmenting the captured image according to the plurality of ranges of colour wavelength by dividing said capture image according to each range of colour wavelength;
defining a path from each said segmented captured image, said path comprising a plurality of path segments wherein each path segment corresponds to a colour wavelength range;
selecting each path segment of the plurality of path segments;
selecting the laser of the exchangeable multi-laser head according to the colour wavelength corresponding to each selected path segment;
removing the tattoo or scar by receiving a user trigger input for shooting the exchangeable multi-laser head into a laser spot regularly spaced along each selected path segment.

It is also disclosed the use of the device and the method for oncological applications, in particular breast reconstruction.

A positive relationship, or varying positively with, defines a relationship between two variables in which as one increases, the other also increases; and reversely as one decreases, the other also decreases.

Impedance control is a generalization of stiffness control. It establishes a dynamical relationship between the position of the laser head and the force, and provide a manipulator control in free-space and in compliant motion with environmental contact. There are different ways of implementing impedance control, namely impedance control and admittance control.

Mechanical impedance referrers to a physical system that accepts motion inputs and yields force outputs.

Mechanical admittance is defined as a physical system that accepts force inputs and yields motion outputs.

Mechanical impedance can have a spring constant component and a damping constant component. A "spring constant" can be defined as a proportionality constant that defines the force output for a tension or compression of the spring. A "damping constant" can also be defined as a force output for a velocity input.

### BRIEF DESCRIPTION OF THE DRAWINGS

The following figures provide preferred embodiments for illustrating the disclosure and should not be seen as limiting the scope of invention.
**Figure 1****:** Schematic representation according to an embodiment of the disclosure of main device modules.
**Figure 2****:** Schematic representation according to an embodiment of the disclosure of further device modules.
**Figure 3****:** Schematic representation according to an embodiment of the disclosure of an automatic laser pointer switch system.
**Figure 4****:** Schematic representation according to an embodiment of the disclosure of an automatic laser pointer switch system using a rotatable arrangement.
**Figure 5****:** Schematic representation according to an embodiment of the disclosure of an automatic laser pointer switch system using a linear axis like concept.
**Figure 6****:** Schematic representation according to an embodiment of the disclosure of a main device workflow.
**Figure 7****:** Schematic representation according to an embodiment of the disclosure of alternatives for positioning operation method.
**Figure 8****:** Schematic representation according to an embodiment of the disclosure of a tattoo treatment planning method.
**Figure 9****:** Schematic representation according to an embodiment of the disclosure of a method for detection of target tattoo regions.
**Figure 10****:** Schematic representation according to an embodiment of the disclosure of a method for tattoo treatment control points selection and trajectory planning.
**Figure 11****:** Schematic representation according to an embodiment of the disclosure of an operation method for user assisted motion.
**Figure 12****:** Schematic representation according to an embodiment of the disclosure of an operation method for automatic motion.
**Figure 13****:** Schematic representation according to an embodiment of the disclosure of an image processing and tracking operation method.
**Figure 14****:** Schematic illustration according to an embodiment of the disclosure of a tracking operation method
**Figure 15****:** Schematic representation according to an embodiment of the disclosure of a trajectory planning method for robot motion control.
**Figure 16****:** Schematic illustration according to an embodiment of the disclosure of an operation method for frame-by-frame trajectory planning for robot motion control.
**Figure 17****:** Schematic representation according to an embodiment of the disclosure of an operation method for robot motion control.
**Figure 18****:** Schematic illustration according to an embodiment of the disclosure of a method for the identification of the control points and treatment trajectory between consecutive treatments.

### DETAILED DESCRIPTION

The present disclosure relates to a laser device and a method for operating said device for laser tattoo or scar removal treatments, preferably for scars after oncological surgery, more preferably form scars after mastectomy and breast reconstruction. Optionally, the device and method can be used for applying or removing pigments for skin reconstruction for oncological patients, preferably for the applying or removal of pigmented skin that form the areola of a breast. The areola is the pigmented area on the breast around the nipple, which can range from pink to red to brown to dark brown or nearly black. Breast reconstruction is a surgery to recreate breasts after mastectomy. In some cases, it is needed to reshape the opposed breast (i.e. the healthy breast) or the implant, including the areola of the nipple, to guarantee a good symmetry between the implant and the opposed breast.

According to an embodiment of the disclosure there is a device and method for laser tattoo or scar removal by collaborative motion between an operator and a robotic support, comprising: an exchangeable laser head having a laser beam target line, and a robotic support of said laser head; further comprising, attached to the laser head or to the robotic support where the laser head is supported: a multispectral camera for capturing an image of skin surface images, a mechanism to automatically change the laser beam wavelength, a robotic arm with fiber optics for laser treatment, preferably integrated, one or more handles for handling by the operator, a force sensor for sensing the intensity and direction of force applied to the handle or handles by the operator for controlling the robotic support, and an electronic data processor configured for carrying out the steps comprising: capturing an image of skin surface comprising a tattoo to be treated by the multispectral camera; identifying, from said captured image and proximal to where the laser beam target line intersects the skin surface, the trajectory treatment path of the tattoo or scar to be treated.

The exchangeable laser head can be a linear laser head or a rotatable laser head. Preferably, the exchangeable laser head is an exchangeable rotatable laser head.

Different control modes are available:
1) applying, through the robotic support, a transversal force, to the laser head along a transversal direction to said path and towards said path; while allowing freely controllable movement of the robotic support by the operator along the direction of said path.
2) automatic tattoo or scar removal treatment, where the robot automatically applies the optimal transversal force, to the laser head along a transversal direction to said path and towards said path in order to guarantee a smooth and correct tattoo or scar removal treatment.

The disclosure may be divided into two aspects, namely:
1) laser treatment planning method for location and segmentation of tattoo or scar on the patient's skin;
2) real-time laser treatment guidance. A device and method to recognize the laser position on the patient's leg has been developed, later transferring all the planning information into the treatment for a real-time treatment evaluation. The complete treatment setup preferably includes a handheld device that combines both collaborative robot-handling system and a user-friendly monitoring software.

The first aspect includes a method and device for planning the tattoo or scar removal with laser therapy. According to an embodiment of the disclosure there are three camera sensors: (1) RGB images to obtain the tattoo or scar texture; (2) multi-spectral cameras for tattoo or scar enhancement; and (3) 3D sensor to obtain the distance between laser/patient's skin. Since these camera sensors have independent world spaces, an initial fusion strategy was implemented. Here, at least two images of a known plan pattern (i.e. chessboard) are acquired at the same time from all the cameras.

Then, with this information, it is possible to calibrate each camera (e.g. distortion removal) as well as to infer the transformation matrix that correlates each one of them [29], [30]. Initially, the operator freely moves the robotic support and positioned it over the tattoo or scar region so that all the tattoo or scar is visible in the screen. Next, a multispectral light system is used to highlight different tattoos colours (e.g. 532, 580, 650, and 1064 nm). This system comprises an image sensor equipped with different light filters and light sources. From the multispectral images, a Deep Learning (DL) strategy has been used for tattoo identification, and characterization (shape and colour). Here, a convolutional neural network (CNN) architecture that allows a stable performance was designed and further trained.

Although some architectures were already implemented for tattoo or scar segmentation, they just allow one single type of image as input. In this disclosure, it was used both RGB/NIR images as input, improving the robustness of the segmentation method. Finally, since the camera's system has limited FoV, it is necessary to capture multiple images to cover all the skins regions with the desired image quality.

Then, to fuse these set of images into a single one, an image mosaicking strategy is used [31], [32]. From the detected tattoo, it is sub-divided according with the indicated wavelength to be treated (i.e. 532, 1064, 585, 650 nm). Then, treatment shoot control points are defined using a shortest path method, having into account the beam shape and size, is used to find the path that covers the entire tattoo region, minimizing overshooting and treatment time. In the end, the complete planning map of the tattoo removal session will be obtained. Planning trajectories, shoot positions, per different wavelength available. Also, the treatment time and the number of shoots predictions should be computed. From this map the physician can select the regions that are to be treated for each specific treatment session, completing the treatment planning strategy. The latter is used to define a laser treatment guidance.

In the second aspect, a treatment guidance system is disclosed. This system incorporates a handheld gripper that combines a monitoring system based on artificial intelligence with the laser device to obtain real-time patient-specific treatment. Moreover, this gripper also allows to plug and play with a collaborative robot.

The first task focuses on a tracking system to obtain real-time positioning of the laser treatment. For it, RGB/NIR images are continuously recorded and analysed using the tattoo or scar segmentation module. Since each segmentation pattern of tattoos are unique, by fit each with the skin panoramic view using a registration strategy, the positioning of the camera sensors throughout the treatment is obtained. Consequently, all planning information from the first aspect can be transferred to the treatment, obtaining patient-specific laser treatment guidance.

The next task focuses on the development of a continuous monitoring software of the patient's skin. The treatment guidance visualization and evaluation is achieved using a user-friendly interface. Here, the real-time evaluation is shown in a user-friendly interface, where are planned the following views: (1) patient's skin condition; (2) tattoo or scar map over patient's skin; (3) laser position; (4) zoomed view of the tattoo or scar map; (5) zoomed view of patient's skin condition. Furthermore, this interface should recognize when the laser focal point is incorrectly positioned and the limits of the tattoo to treatment (i.e. vessels clearance) or scar to treatment.

When any of these situations arise, the physician must be immediately alerted, thus minimizing side effects related to over-treatment [26]. Finally, laser handling assistance is foreseen using a collaborative robot (i.e. LBR Med), helping the physician to guarantee the optimal relation laser/patient's skin over the treatment session. Initially, all the systems (i.e. cameras, laser) are integrated with the robot's end-effector.

Next, different control strategies has been developed to: 1) keep the distance/angle between laser/patient's skin; 2) force the laser positioning at the path centre when near; 3) disable the laser when outside the tattoo or scar path; and, 4) keep the laser under the limits of treatment. An automatic mode is also possible. Here, instead of an external force from the operator to move the robot, it automatically computed the optimal force to the laser head to guarantee a smooth and correct tattoo or scar removal treatment.

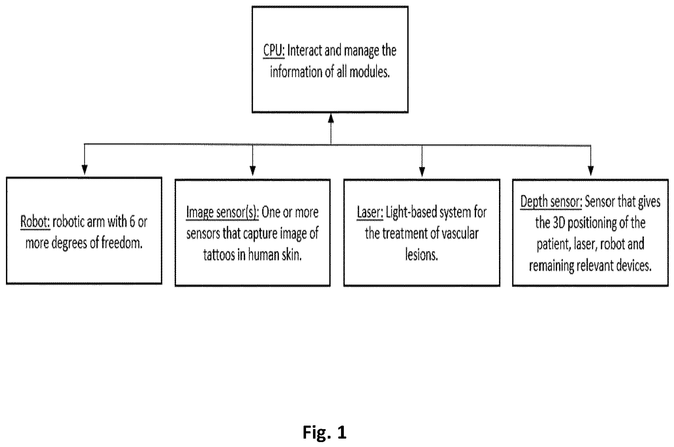

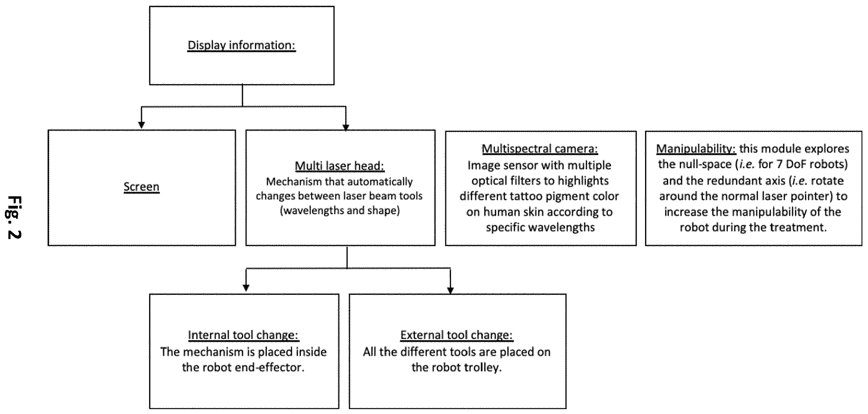

Figures 1 and 2 describes preferred embodiments of the device modules. Figure 1 shows the mandatory modules of the proposed system, while Figure 2 presents some additional options.

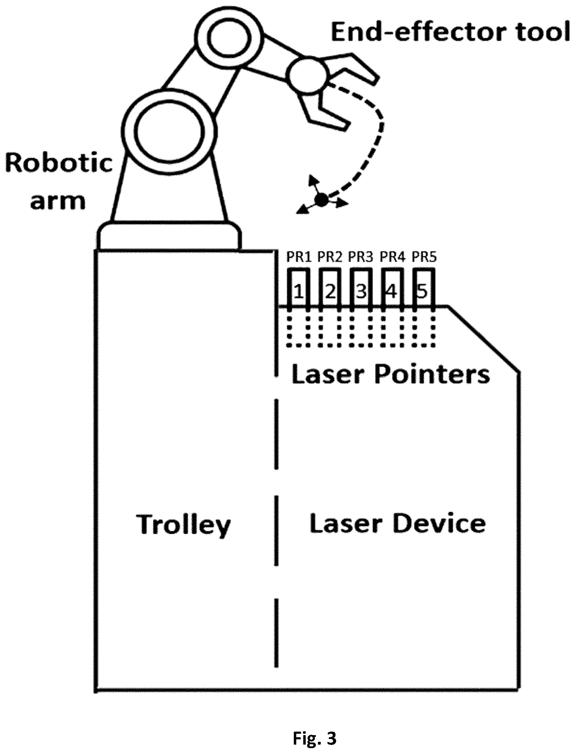

Figure 3 describes a preferred embodiment of the automatic laser pointer switch system. Different laser points are positioned at the laser device. The robotic arm (positioned on a trolley) is positioned near the laser device. The laser pointers positioned (PR1, PR2, PR3, PR4, PR5 or more/less) are calibrated with the robot base. A motor will be controlled by an external electronic device to modify its configuration and consequently to change the laser pointers. A mechanical device (e.g. pressure system) is positioned at the robot gripper to connect the fiber optic and the laser pointer.

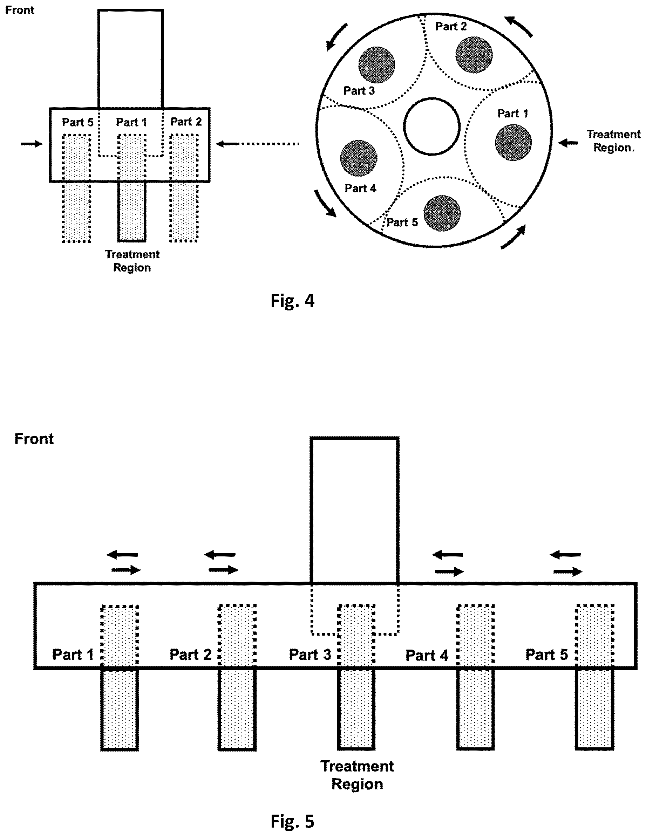

Figure 4 describes a preferred embodiment of the automatic laser pointer switch system using a rotatable concept. Different laser pointers, described as Part 1, Part 2, Part 3, Part 4 and Part 5 or more/less, are positioned on a circular structure. Based on the required wavelength, the structure will rotate to position the target laser pointer in the treatment region. A motor controlled by an electronic device will be used to modify the laser pointers.

Figure 5 describes a preferred embodiment of an automatic laser pointer switch system using a linear axis like concept. Different laser pointers (described as Part 1, Part 2, Part 3, Part 4 and Part 5 or more/less) are positioned on a linear structure. Based on the required wavelength, the structure will slide throughout the different laser pointer options. The central laser pointer is defined as the treatment region.

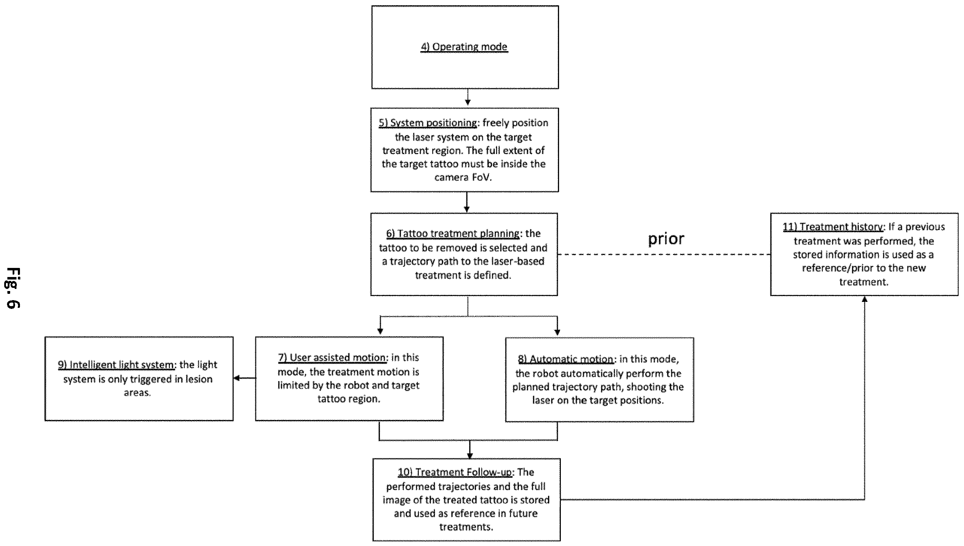

Figure 6 describes a preferred embodiment of the method. A detailed description of the preferred operation mode is presented. First, the system positioning is used to freely move the device into the target region. Then, a treatment planning of the tattoo to be removed will be carried out. From the defined trajectory paths for the treatment, an automatic or userassisted motion will be used to treat the target region or to execute the planned trajectories. A treatment follow-up to monitor the patient evolution is finally applied.

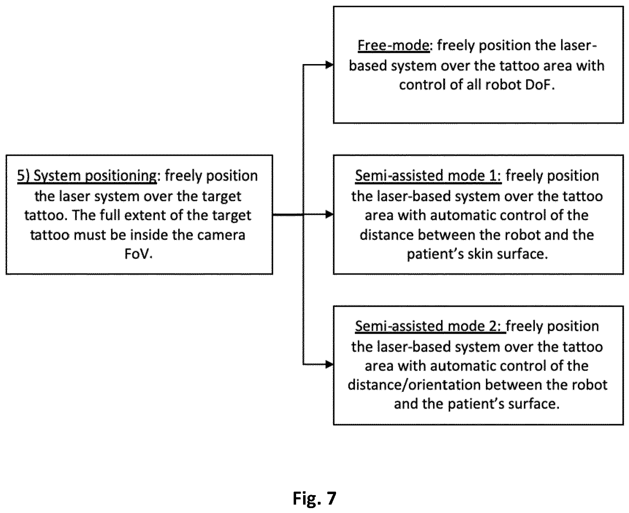

Figure 7 describes alternatives for positioning operation method: 1) Free-mode - freely position the device over the tattoo area with control of all robot degree of freedom (DoF); 2) freely position the device with automatic control of the distance between the robot and the patient's skin; 3) freely position the device with automatic control of the distance and orientation between the robot and the patient's skin.

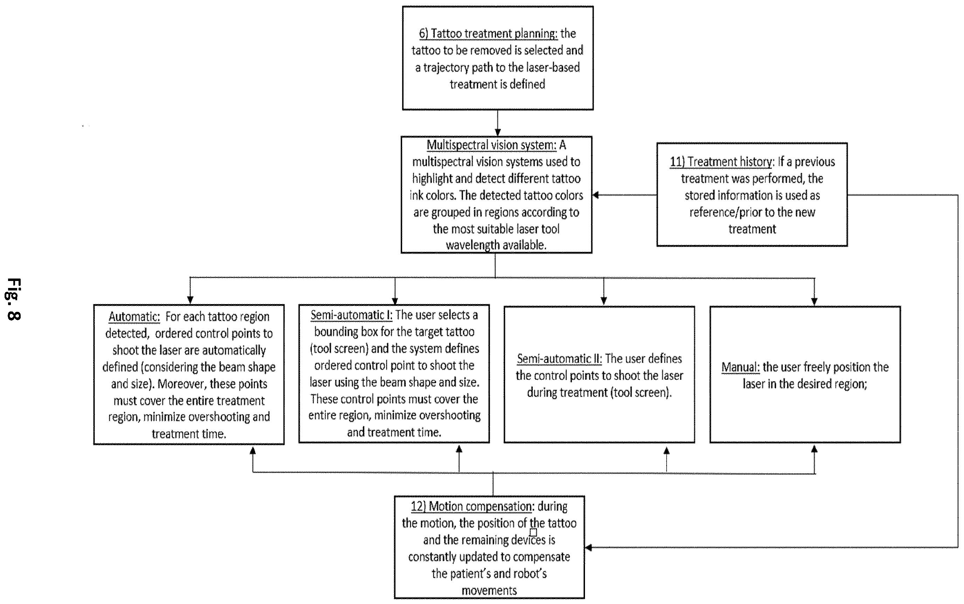

Figure 8 is a preferred embodiment of the treatment planning method. First a multispectral vision system is used to detect and group different tattoo regions colors according with the available laser wavelengths. Depending on level of collaboration (i.e. automatic, semi-automatic mode I, semi-automatic mode II e manual) selected by the operator, the trajectories and the control points for the treatment are defined.

In the automatic mode, for each tattoo region detected, ordered control points to shoot the laser are automatically defined (considering the beam shape and size). Moreover, these points must cover the entire treatment region, minimize overshooting and treatment time. In the semi-automatic mode, the user selects a bounding box for the target tattoo (tool screen) and the system defines ordered control point to shoot the laser using the beam shape and size or the user defines the control points to shoot the laser during treatment (tool screen). In manual mode, the user freely positions the laser in the desired region.

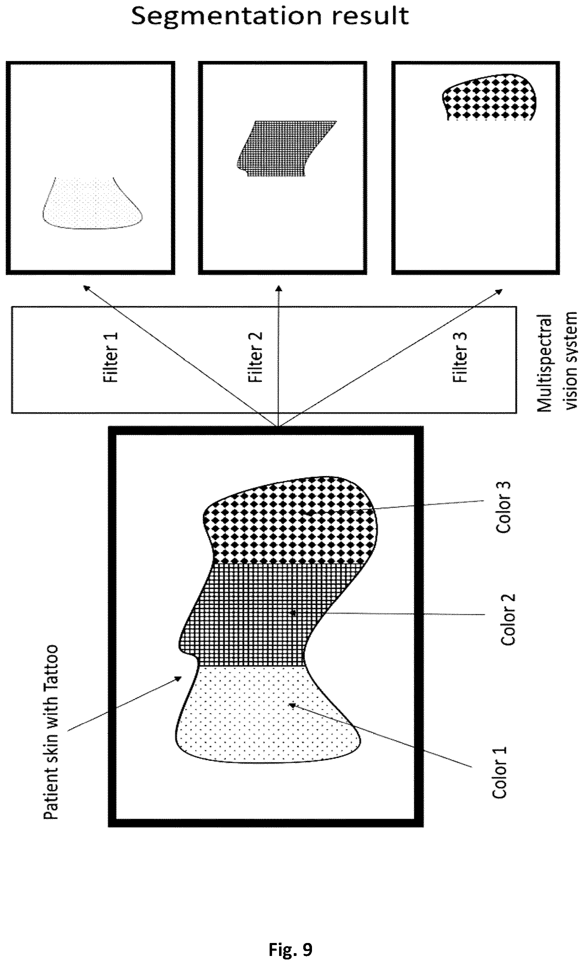

Figure 9 is a preferred embodiment of the method for detection of target tattoo regions. Target tattoos are divided in different regions, designated by treatment phases, according to the available laser wavelengths. Different optical filters or optical filters arrays are used to highlight specific light wavelengths. Then, from each highlighted image, segmentation methods are used to detect the regions to be treated with each specific laser wavelength.

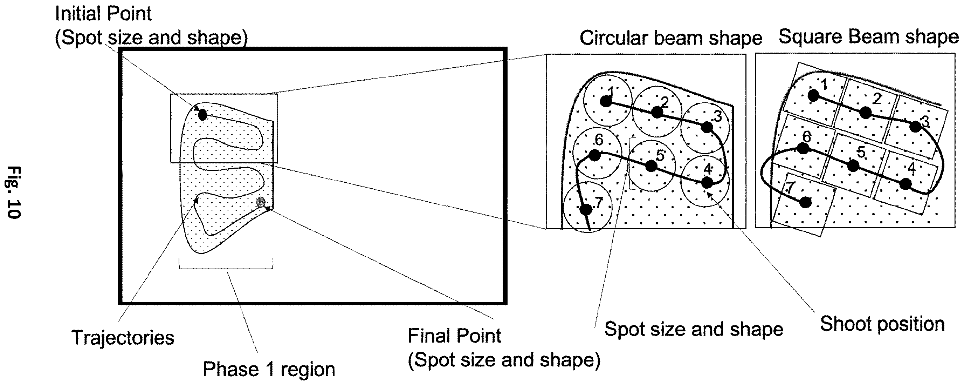

Figure 10 is a preferred embodiment of the method for control points selection and trajectory planning. For each treatment phase, the shortest path is defined. Here, control points are placed in the tattoo (covering its full extent) depending on the spot size. Then, a shortest trajectory path is defined to the robot in order to pass in all points and avoid discontinuity problems on the velocity/acceleration profile of the robot.

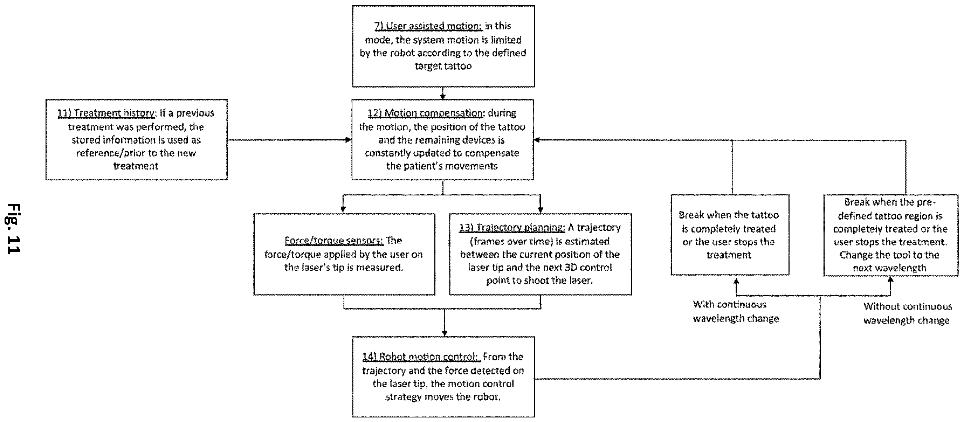

Figure 11 is a preferred embodiment of the method, when there is a collaborative motion between the operator and the robotic support.

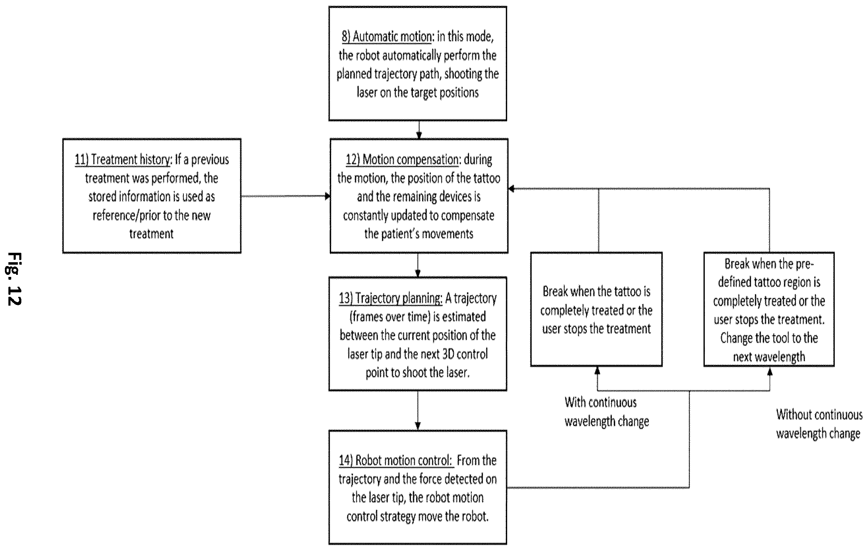

Figure 12 is a preferred embodiment of the method, when the device is used in automatic motion.

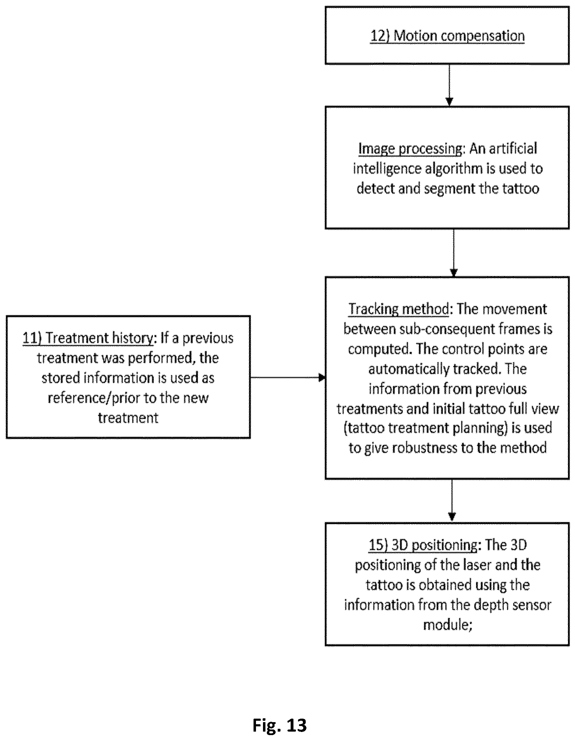

Figure 13 is a preferred embodiment of the image processing and tracking operation method.

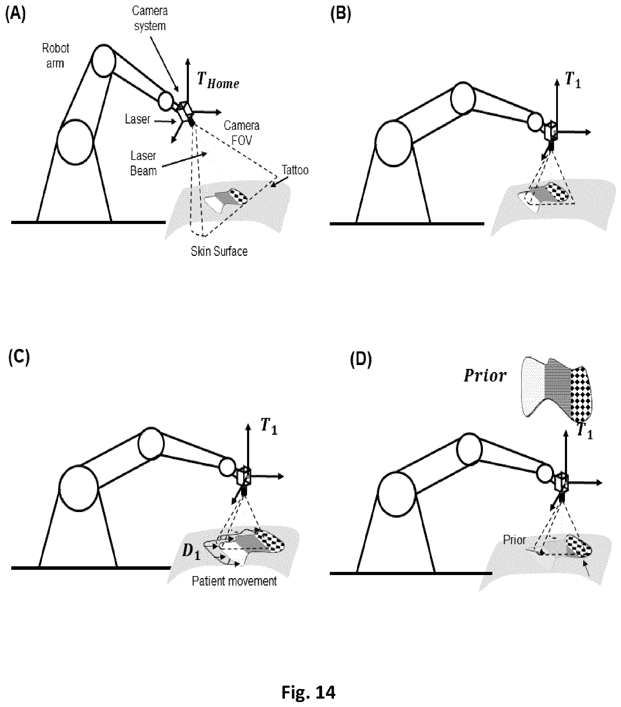

Figure 14 is a preferred embodiment of the tracking operation method. Tracking strategy: (A) Visualization of the entire tattoo and definition of a general coordinate system using the robot configuration. (B) Robot movement to the first treatment point. The variation of the robot configuration respectively to the previous state is used to rigidly track the target point. Note that, a previous calibration between the camera and the robot was established. (C) After rigidly aligning the different images (previous treatment moment and the current one), an image tracking algorithm is applied to remove possible misalignments caused by possible patient movements during the treatment. (D) During the treatment, the entire tattoo shape and the planned trajectories are used as prior (transparent region) to prevent tracking errors caused by image occlusions.

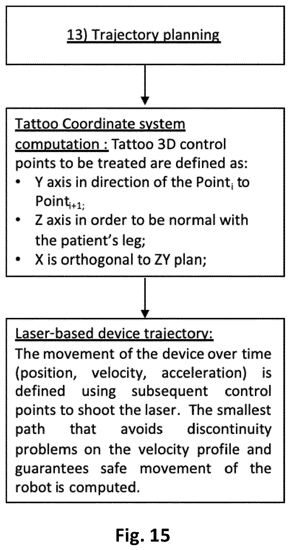

Figure 15 is a preferred embodiment of the trajectory planning method for robotic motion control.

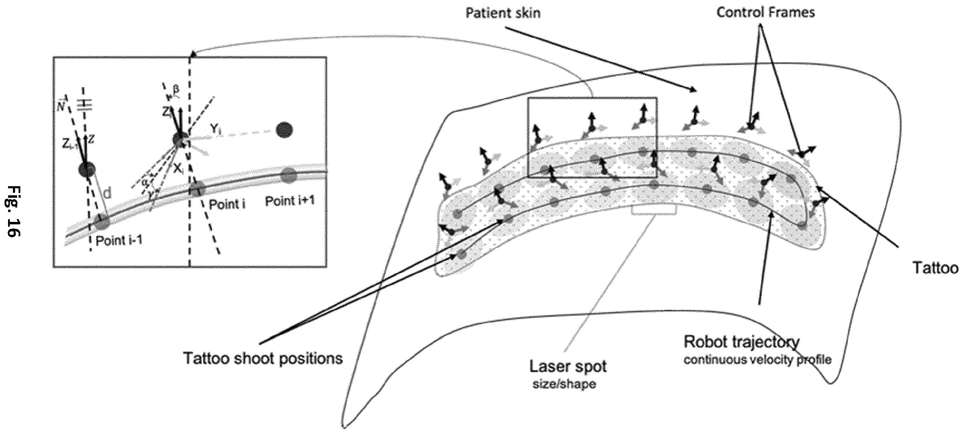

Figure 16 is a preferred embodiment of the method for frame-by-frame trajectory planning for robotic motion control.

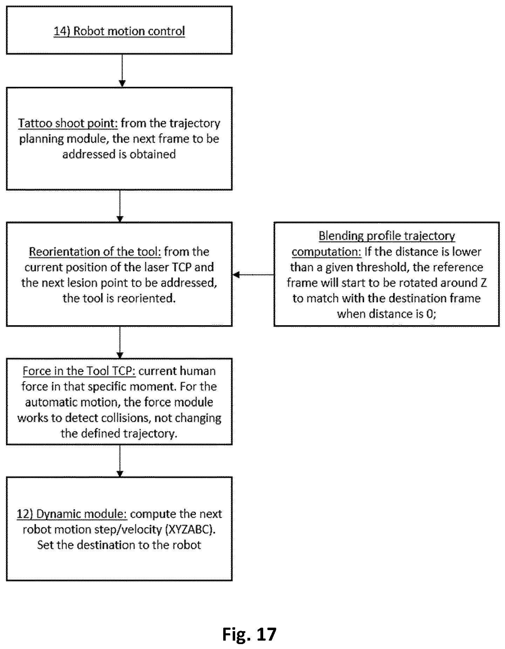

Figure 17 is a preferred embodiment of the method for robotic motion control.

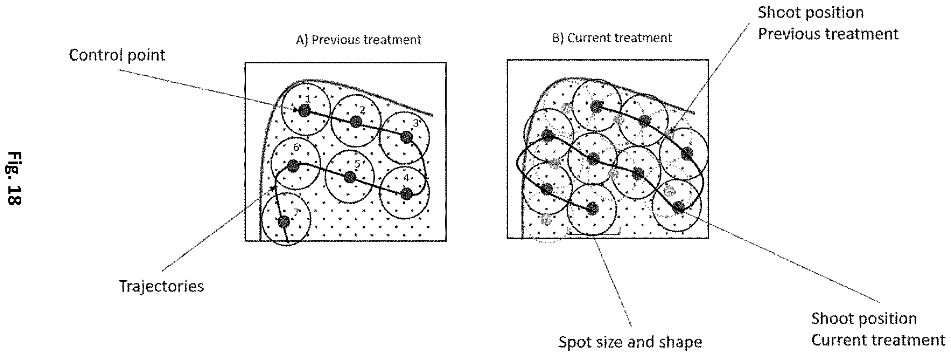

Figure 18 is a preferred embodiment of the method for the identification of the control points and treatment trajectory between consecutive treatments. (A) Previous treatment trajectory. (B) Considering the previous treatment trajectory (dashed circles), a new set of control points (black circles) are estimated in intermediate positions of the previous control points (grey circles) maximizing the treatment area between consecutive treatment sessions.

The term "comprising" whenever used in this document is intended to indicate the presence of stated features, integers, steps, components, but not to preclude the presence or addition of one or more other features, integers, steps, components or groups thereof.

It is to be appreciated that certain embodiments of the disclosure as described herein may be incorporated as code (e.g., a software algorithm or program) residing in firmware and/or on computer useable medium having control logic for enabling execution on a computer system having a computer processor, such as any of the servers described herein. Such a computer system typically includes memory storage configured to provide output from execution of the code which configures a processor in accordance with the execution. The code can be arranged as firmware or software, and can be organized as a set of modules, including the various modules and algorithms described herein, such as discrete code modules, function calls, procedure calls or objects in an object-oriented programming environment. If implemented using modules, the code can comprise a single module or a plurality of modules that operate in cooperation with one another to configure the machine in which it is executed to perform the associated functions, as described herein.

The disclosure should not be seen in any way restricted to the embodiments described and a person with ordinary skill in the art will foresee many possibilities to modifications thereof. The above described embodiments are combinable. The following claims further set out particular embodiments of the disclosure.

## Claims

1. Device for laser removal of a tattoo or a scar extending along a pre-defined skin area, comprising:
a skin treatment multi-laser exchangeable head having at least one laser beam target line, and
a robotic support for supporting said laser head;
further comprising, attached to the exchangeable multi-laser head or to the robotic support where the exchangeable multi-laser head is supported:
a multispectral camera for capturing skin surface images having plurality of ranges of colour wavelength of the skin surface image, each range corresponding to a laser of the exchangeable multi-laser head,
a mechanism configured to automatically exchange the laser head for changing between laser beams of the exchangeable multi-laser head,
a robotic arm comprising fibre optics;
one or more handles for handling by the operator,
a force sensor for sensing the intensity and direction of force applied to the handle or handles by the operator for controlling the robotic support, and
an electronic data processor configured for carrying out the steps comprising:
capturing an image of skin surface comprising the tattoo to be removed, by the multispectral camera;
segmenting the captured image according to the plurality of ranges of colour wavelength by dividing said capture image according to each range of colour wavelength;
defining a path from each said segmented captured image, said path comprising a plurality of path segments wherein each path segment corresponds to a colour wavelength range;
selecting each path segment from the plurality of defined path segments;
selecting the laser of the exchangeable multi-laser head according to the colour wavelength corresponding to each selected path segment;
removing the tattoo or the scar by receiving a user trigger input for shooting the exchangeable multi-laser head into a laser spot regularly spaced along each selected path segment.

2. Device for laser removal of a tattoo or a scar according to the previous claim wherein said path further comprises a plurality of connecting curve segments, each curve between two segmented paths wherein the curve connecting paths has been obtained by using the maximum speed and acceleration of the robot arm as defined for a safe treatment operation of the robot arm.

3. Device for laser removal of a tattoo or a scar according to any of the previous claim wherein the electronic data processor is configured for carrying out the step of applying, through the robotic support, a transversal force, to the exchangeable multi-laser head along a transversal direction to said path and towards said selected path while allowing freely controllable movement of the robotic support along the direction of said selected path.

4. Device for laser removal of a tattoo or a scar according to claims 1-2 wherein the electronic data processor is configured for carrying out the step of applying, automatically by the robot, a transversal direction to the path and towards said path of the exchangeable multi-laser head, preferably the transversal direction is parallel to the skin surface.

5. Device for laser removal of a tattoo or a scar according to the previous claim wherein the electronic data processor is further configured for maintaining, through the robotic support, a first transversal distance between said path and where the laser beam target line intersects the skin surface, wherein said first transversal distance varies positively with a first force as measured by said force sensor along said transversal direction parallel to the skin surface, preferably said first transversal distance is directly proportional to said force.

6. Device for laser removal of a tattoo or a scar according to any of the previous claims wherein the electronic data processor is further configured for maintaining, through the robotic support, a second transversal distance between said laser head and skin surface, along a further transversal direction, wherein said further transversal direction is perpendicular to the skin surface, and
wherein said second transversal distance, offset by a predetermined amount of distance, varies positively with a second force as measured by said force sensor along said further transversal direction perpendicular to the skin surface, preferably said second transversal distance is directly proportional to said further force.

7. Device for laser removal of a tattoo or a scar according to claims 4 wherein the proportionality constant of the first transversal distance relative to the first force and the proportionality constant of the second transversal distance relative to the second force are different,
in particular the proportionality constant of the first transversal distance relative to the first force is higher than the proportionality constant of the second transversal distance relative to the second force.

8. Device for laser removal of a tattoo or a scar according to any of the claims 1-5, wherein the electronic data processor is further configured for applying, through the robotic support, a further force to said laser head along a further transversal direction to said path and towards said path, wherein said further transversal direction is perpendicular to the skin surface, such that a second transversal distance between multi-laser exchangeable head and skin surface is kept constant and equal to a predetermined amount and/or wherein said electronic data processor is further configured for applying a speed dampening to the movement of the robotic support which is inversely related to the speed of the multi-laser exchangeable head.

9. Device for laser removal of a tattoo or a scar according to any of the previous claims, where the electronic data processor is further configured to shoot the laser when the distance between the current position and the control point is equal or less to a predefined constant, wherein this distance is defined to guarantee the safeness of the treatment and/or wherein the electronic data processor is further configured to automatically change between control points after the current control point be treated.

10. Device for laser removal of a tattoo or a scar according to any of the previous claims, wherein the electronic data processor is further configured for applying through the robotic support a torque to said laser head, such that the angle between the beam of the laser head and skin surface is kept perpendicular and/or wherein the electronic data processor is further configured for applying, through the robotic support, a force to said laser head longitudinally to said path such that the multi-laser exchangeable head is freely movable along said path by the operator using said handle or handles and/or wherein the electronic data processor is further configured for applying through the robotic support a force to said laser head along a transversal direction to said path and towards said path, wherein said transversal direction is perpendicular to the skin surface, such that the distance between laser head and skin surface is kept constant and equal to a predetermined amount minus a predetermined tattoo depth.

11. Device for laser removal of a tattoo or a scar according to any of the previous claims wherein the force sensor is mounted to sense intensity and direction of the force applied by the operator directly to the handle or handles, or
mounted to sense intensity and direction of the force applied by the operator indirectly to the handle or handles by sensing the intensity and direction of force applied by the operator to a joystick mounted on the handle or handles.

12. Device for laser removal of a tattoo according to any of the previous claims, wherein the electronic data processor is further configured for combining the NIR and VIS captured images.

13. Device for laser removal of a tattoo or a scar according to any of the previous claims, comprising 3 or more laser rangefinders arranged at, or in the vicinity of, the laser head for determining the distance between the laser head and the skin surface, or for determining the angle between the laser head beam and the skin surface or for their combination and/or comprising 3 or more laser rangefinders arranged at, or in the vicinity of, the multi-laser head in order to overlay the rangefinder laser spots in the captured skin surface image for determining pixel size of the skin surface image or for aligning the captured skin surface image with the multi- laser exchangeable head or for their combination.

## Patentansprüche

1. Vorrichtung zur Laserentfernung einer Tätowierung oder einer Narbe, die sich entlang eines vordefinierten Hautbereichs erstreckt, umfassend:
einen austauschbaren Mehrfach-Laserkopf zur Hautbehandlung mit mindestens einem Ziellinien-Laserstrahl, und
eine Roboterhalterung zum Halten des genannten Laserkopfes;
ferner umfassend, folgende an dem austauschbaren Mehrfach-Laserkopf oder an der Roboterhalterung, an dem der austauschbare Mehrfach-Laserkopf befestigt ist, angebrachte Komponenten:
eine Multispektralkamera zum Erfassen von Bildern der Hautoberfläche mit einer Vielzahl von Wellenlängenbereichen der Farben des Bildes der Hautoberfläche, wobei jeder Bereich einem Laser des austauschbaren Mehrfach-Laserkopfes entspricht,
einen Mechanismus, der so konfiguriert ist, dass er den Laserkopf automatisch austauscht, um zwischen den Laserstrahlen des austauschbaren Mehrfach-Laserkopfes zu wechseln, einen Roboterarm mit Lichtwellenleitern;
einen oder mehrere Griffe für die Bedienung durch den Bediener,
einen Kraftaufnehmer, der die Größe und Richtung der vom Bediener auf den Handgriff oder die Handgriffe ausgeübten Kraft erfasst, um die Roboterhalterung zu steuern, und
einen elektronischen Datenprozessor, der so konfiguriert ist, dass er folgende Schritte ausführt:
Erfassen eines Bildes der Hautoberfläche mit der zu entfernenden Tätowierung mit der Multispektralkamera;
Segmentieren des erfassten Bildes entsprechend der Vielzahl der Wellenlängenbereiche der Farben durch Aufteilen des genannten erfassten Bildes entsprechend des jeweiligen Wellenlängenbereichs der Farbe;
Bestimmen einer Strecke für jedes der genannten segmentierten erfassten Bilder, wobei die genannte Strecke eine Vielzahl von Streckensegmenten umfasst, wobei jedes Streckensegmenten einem Wellenlängenbereich der Farbe entspricht;
Auswählen jedes Streckensegmentens aus der Vielzahl der bestimmten Streckensegmente;
Auswählen des Lasers des austauschbaren Mehrfach-Laserkopfes entsprechend der Wellenlänge der Farbe, die jedem ausgewählten Streckensegment entspricht;
Entfernen der Tätowierung oder der Narbe durch Empfang einer Triggereingabe des Benutzers, um den austauschbaren Mehrfach-Laserkopf in einem gleichmäßig beabstandeten Brennfleck des Lasers entlang jedes ausgewählten Streckensegments zu aktivieren.

2. Vorrichtung zur Laserentfernung einer Tätowierung oder einer Narbe nach dem vorangehenden Anspruch, wobei die genannte Strecke ferner eine Vielzahl von verbindenden Kurvensegmenten umfasst, wobei jede Kurve zwischen zwei segmentierten Strecken liegt, wobei die die Strecken verbindende Kurve unter Verwendung der maximalen Geschwindigkeit und Beschleunigung des Roboterarms erreicht wird, die so eingestellt sind, dass sie einen sicheren Behandlungsbetrieb des Roboterarms ermöglichen.

3. Vorrichtung zur Laserentfernung einer Tätowierung oder einer Narbe nach einem der vorangehenden Ansprüche, wobei der elektronische Datenprozessor so konfiguriert ist, dass er den Schritt des Anlegens einer Querkraft durch die Roboterhalterung auf den austauschbaren Mehrfach-Laserkopf entlang einer Richtung quer zu der genannten Strecke und in Richtung der genannten ausgewählten Strecke ausführt, während er eine frei steuerbare Bewegung der Roboterhalterung entlang der Richtung der genannten ausgewählten Strecke ermöglicht.

4. Vorrichtung zur Laserentfernung einer Tätowierung oder einer Narbe nach einem der Ansprüche 1-2, wobei der elektronische Datenprozessor so konfiguriert ist, dass er den Schritt des automatischen Anlegens einer Querrichtung an die Strecke und in Richtung der genannten Strecke des austauschbaren Mehrfach-Laserkopfes durch den Roboter ausführt, wobei die Querrichtung bevorzugt parallel zur Hautoberfläche verläuft.

5. Vorrichtung zur Laserentfernung einer Tätowierung oder einer Narbe nach dem vorangehenden Anspruch, wobei der elektronische Datenprozessor ferner so konfiguriert ist, dass er durch die Roboterhalterung einen ersten Querabstand zwischen der genannten Strecke und der Stelle, an der die Ziellinie des Laserstrahls die Hautoberfläche schneidet, aufrechterhält, wobei der erste genannte Querabstand positiv mit einer ersten Kraft variiert, die von dem genannten Kraftaufnehmer entlang der genannten Querrichtung parallel zur Hautoberfläche gemessen wird, wobei der erste genannte Querabstand bevorzugt direkt proportional zu der genannten Kraft ist.

6. Vorrichtung zur Laserentfernung einer Tätowierung oder einer Narbe nach einem der vorangehenden Ansprüche, wobei der elektronische Datenprozessor ferner so konfiguriert ist, dass er durch die Roboterhalterung einen zweiten transversalen Abstand zwischen dem genannten Laserkopf und der Hautoberfläche entlang einer weiteren transversalen Richtung aufrechterhält, wobei die genannte weitere transversale Richtung senkrecht zur Hautoberfläche ist, und
wobei der genannte zweite transversale Abstand, der um eine vorbestimmte Abstandsgröße versetzt ist, positiv mit einer zweiten Kraft variiert, die von dem genannten Kraftaufnehmer entlang der genannten weiteren transversalen Richtung senkrecht zur Hautoberfläche gemessen wird, wobei der genannte zweite transversale Abstand bevorzugt direkt proportional zu der genannten weiteren Kraft ist.

7. Vorrichtung zur Laserentfernung einer Tätowierung oder einer Narbe nach Anspruch 4, wobei die Proportionalitätskonstante des ersten Querabstands relativ zur ersten Kraft und die Proportionalitätskonstante des zweiten Querabstands relativ zur zweiten Kraft unterschiedlich sind,
wobei insbesondere die Proportionalitätskonstante des ersten Querabstands relativ zur ersten Kraft größer ist als die Proportionalitätskonstante des zweiten Querabstands relativ zur zweiten Kraft.

8. Vorrichtung zur Laserentfernung einer Tätowierung oder einer Narbe nach einem der Ansprüche 1-5, wobei der elektronische Datenprozessor ferner so konfiguriert ist, dass er über die Roboterhalterung eine weitere Kraft auf den genannten Laserkopf entlang einer weiteren Querrichtung zu der genannten Strecke und in Richtung der genannten Strecke ausübt, wobei die genannte weitere Querrichtung senkrecht zur Hautoberfläche verläuft, so dass ein zweiter Querabstand zwischen dem austauschbaren Mehrfach-Laserkopf und der Hautoberfläche konstant gehalten wird und gleich einer vorbestimmten Größe ist, und/oder wobei der genannte elektronische Datenprozessor ferner so konfiguriert ist, dass er eine Geschwindigkeitsdämpfung auf die Bewegung der Roboterhalterung anwendet, die umgekehrt zur Geschwindigkeit des austauschbaren Mehrfach-Laserkopfes ist.

9. Vorrichtung zur Laserentfernung einer Tätowierung oder einer Narbe nach einem der vorangehenden Ansprüche, wobei der elektronische Datenprozessor ferner so konfiguriert ist, dass er den Laser auslöst, wenn der Abstand zwischen der aktuellen Position und dem Kontrollpunkt gleich oder kleiner als eine vordefinierte Konstante ist, wobei dieser Abstand so bestimmt ist, dass die Sicherheit der Behandlung sichergestellt ist, und/oder wobei der elektronische Datenprozessor ferner so konfiguriert ist, dass er automatisch zwischen Kontrollpunkten wechselt, nachdem der aktuelle Kontrollpunkt behandelt wurde.

10. Vorrichtung zur Laserentfernung einer Tätowierung oder einer Narbe nach einem der vorangehenden Ansprüche, wobei der elektronische Datenprozessor ferner so konfiguriert ist, dass er über die Roboterhalterung ein Drehmoment auf den genannten Laserkopf ausübt, so dass der Winkel zwischen dem Strahl des Laserkopfes und der Hautoberfläche senkrecht gehalten wird, und/oder wobei der elektronische Datenprozessor ferner so konfiguriert ist, dass er über die Roboterhalterung eine Kraft auf den genannten Laserkopf in Längsrichtung der genannten Strecke ausübt, so dass der austauschbare Mehrfach-Laserkopf durch den Bediener unter Verwendung des genannten Griffs oder der Griffe frei entlang der genannten Strecke bewegt werden kann, und/oder wobei der elektronische Datenprozessor ferner so konfiguriert ist, dass er durch die Roboterhalterung eine Kraft auf den genannten Laserkopf entlang einer Querrichtung zu der genannten Strecke und in Richtung der genannten Strecke ausübt, wobei die genannte Querrichtung senkrecht zu der Hautoberfläche ist, so dass der Abstand zwischen dem Laserkopf und der Hautoberfläche konstant gehalten wird und gleich einer vorbestimmten Größe minus einer vorbestimmten Tätowiertiefe ist.

11. Vorrichtung zur Laserentfernung einer Tätowierung oder einer Narbe nach einem der vorangehenden Ansprüche, wobei der Kraftaufnehmer so angebracht ist, dass er die Intensität und Richtung der von dem Bediener direkt auf den Griff oder die Griffe ausgeübten Kraft erfasst, oder
so angebracht ist, dass er die Intensität und Richtung der von dem Bediener indirekt auf den Griff oder die Griffe ausgeübten Kraft erfasst, indem er die Intensität und Richtung der von dem Bediener auf einen an dem Griff oder den Griffen angebrachten Joystick ausgeübten Kraft erfasst.

12. Vorrichtung zur Laserentfernung einer Tätowierung nach einem der vorangehenden Ansprüche, wobei der elektronische Datenprozessor ferner so konfiguriert ist, dass er die erfassten NIR- und VIS-Bilder kombiniert.

13. Vorrichtung zur Laserentfernung einer Tätowierung oder einer Narbe nach einem der vorangehenden Ansprüche, umfassend 3 oder mehr Laser-Entfernungsmessern, die am oder in der Nähe des Laserkopfes angeordnet sind, um den Abstand zwischen dem Laserkopf und der Hautoberfläche oder den Winkel zwischen dem Strahl des Laserkopfs und der Hautoberfläche oder deren Kombination zu bestimmen, und/oder umfassend 3 oder mehr Laser-Entfernungsmessern, die am oder in der Nähe des Mehrfach-Laserkopfes angeordnet sind, um die Entfernungsmesser für den Brennfleck des Lasers in dem erfassten Bild der Hautoberfläche zu überlagern, um die Pixelgröße des Bildes der Hautoberfläche zu bestimmen oder um das erfasste Bild der Hautoberfläche mit dem austauschbaren Mehrfach-Laserkopf auszurichten, oder diese zu kombinieren.

## Revendications

1. Dispositif pour l'élimination au laser d'un tatouage ou d'une cicatrice s'étendant le long d'une zone de peau prédéfinie, comprenant :
une tête interchangeable multi-laser de traitement de peau ayant au moins une ligne de mire de faisceau laser, et
un support robotique pour soutenir ladite tête de laser ;
comprenant également, attachés à la tête multi-laser interchangeable ou au support robotique où la tête multi-laser interchangeable est soutenue :
une caméra multispectrale pour capturer des images de la surface de la peau ayant une pluralité de gammes de longueurs d'onde de couleur de l'image de la surface de la peau, chaque gamme correspondant à un laser de la tête multi-laser interchangeable,
un mécanisme configuré pour changer automatiquement la tête de laser pour échanger les faisceaux de laser de la tête multi-laser interchangeable,
un bras robotique comprenant des fibres optiques ;
une ou plusieurs poignées destinées à être utilisées par l'opérateur,
un capteur de force pour capter l'intensité et la direction de la force appliquée à la poignée ou aux poignées par l'opérateur pour contrôler le support robotique, et
un traiteur de données électronique configuré pour exécuter les étapes comprenant :
capturer une image de la surface de la peau comprenant le tatouage à éliminer, à l'aide de la caméra multispectrale ;
segmenter l'image capturée en fonction de la pluralité de gammes de longueurs d'onde de couleur en divisant ladite image capturée en fonction de chaque gamme de longueurs d'ondes de couleur ;
définir une trajectoire à partir de chacune desdites images capturées segmentées, ladite trajectoire comprenant une pluralité de segments de trajectoire dans laquelle chaque segment de trajectoire correspond à une gamme de longueurs d'ondes de couleur ;
sélectionner chaque segment de trajectoire à partir de la pluralité de segments de trajectoire définis ;
sélectionner le laser de la tête multi-laser interchangeable en fonction de la longueur d'ondes de couleur correspondant à chaque segment de trajectoire sélectionné ;
éliminer le tatouage ou la cicatrice en recevant un signal de déclenchement venu de l'utilisateur pour actionner la tête multi-laser interchangeable vers un point laser régulièrement espacé le long de chaque segment de trajectoire sélectionné.

2. Dispositif pour l'élimination au laser d'un tatouage ou d'une cicatrice selon la revendication précédente dans lequel ladite trajectoire comprend également une pluralité de segments courbes reliés, chaque courbe étant entre deux trajectoires segmentées, dans lequel la courbe reliant les trajectoires a été obtenue en utilisant la vitesse et l'accélération maximales du bras robotique telles que définies pour une opération de traitement du bras robotique sûre.

3. Dispositif pour l'élimination au laser d'un tatouage ou d'une cicatrice selon l'une quelconque des revendications précédentes dans lequel le traiteur données électronique est configuré pour exécuter l'étape consistant à appliquer, à travers le support robotique, une force transversale à la tête multi-laser interchangeable le long d'une direction transversale à ladite trajectoire et vers ladite trajectoire sélectionnée tout en permettant le mouvement librement contrôlable du support robotique le long de la direction de ladite trajectoire sélectionnée.

4. Dispositif pour l'élimination au laser d'un tatouage ou d'une cicatrice selon les revendications 1-2 dans lequel le traiteur de données électronique est configuré pour exécuter l'étape consistant à appliquer, automatiquement à l'aide du robot, une direction transversale à la trajectoire et vers ladite trajectoire de la tête multi-laser interchangeable, la direction transversale étant préférablement parallèle à la surface de la peau.

5. Dispositif pour l'élimination au laser d'un tatouage ou d'une cicatrice selon la revendication précédente dans lequel le traiteur de données électronique est également configuré pour maintenir, à travers le support robotique, une première distance transversale entre ladite trajectoire et l'endroit où la ligne de mire du faisceau laser intersecte la surface de la peau, dans lequel ladite première distance transversale varie positivement avec une première force telle que mesurée par ledit capteur de force le long de ladite direction transversale parallèle à la surface de la peau, ladite première distance transversale étant préférablement directement proportionnelle à ladite force.

6. Dispositif pour l'élimination au laser d'un tatouage ou d'une cicatrice selon l'une quelconque des revendications précédentes dans lequel le traiteur de données électronique est également configuré pour maintenir, à travers le support robotique, une seconde distance transversale entre ladite tête de laser et la surface de la peau, le long d'une direction transversale supplémentaire, dans lequel ladite direction transversale supplémentaire est perpendiculaire à la surface de la peau, et
dans lequel ladite seconde distance transversale, compensée par une valeur prédéterminée de distance, varie positivement avec une seconde force telle que mesurée par ledit capteur de force le long de ladite direction transversale supplémentaire perpendiculaire à la surface de la peau, ladite seconde distance transversale étant préférablement directement proportionnelle à ladite force supplémentaire.

7. Dispositif pour l'élimination au laser d'un tatouage ou d'une cicatrice selon la revendication 4 dans lequel la constante de proportionnalité de la première distance transversale par rapport à la première force et la constante de proportionnalité de la seconde distance transversale par rapport à la seconde force sont différentes,
la constante de proportionnalité de la première distance transversale par rapport à la première force étant particulièrement supérieure à la constante de proportionnalité de la seconde distance transversale par rapport à la seconde force.

8. Dispositif pour l'élimination au laser d'un tatouage ou d'une cicatrice selon l'une quelconque des revendications 1-5, dans lequel le traiteur de données électronique est également configuré pour appliquer, à l'aide du support robotique, une force supplémentaire sur ladite tête de laser le long d'une direction transversale supplémentaire par rapport à ladite trajectoire et vers ladite trajectoire, dans lequel ladite direction transversale supplémentaire est perpendiculaire à la surface de la peau, telle qu'une seconde distance transversale entre la tête multi-laser interchangeable et la surface de la peau soit maintenue constante et égale à une valeur prédéterminée et/ou dans lequel ledit traiteur de données électronique est également configuré pour appliquer un amortissement de vitesse au mouvement du support robotique qui est lié de manière inverse à la vitesse de la tête multi-laser interchangeable.

9. Dispositif pour l'élimination au laser d'un tatouage ou d'une cicatrice selon l'une quelconque des revendications précédentes, dans lequel le traitement de données électronique est également configuré pour actionner le laser lorsque la distance entre la position actuelle et le point de contrôle est égale ou inférieure à une constante prédéfinie, dans lequel cette distance est définie pour garantir la sûreté du traitement et/ou dans lequel le traiteur de données électronique est également configuré pour changer automatiquement entre des points de contrôle après que le point de contrôle actuel soit traité.

10. Dispositif pour l'élimination au laser d'un tatouage ou d'une cicatrice selon l'une quelconque des revendications précédentes, dans lequel le traiteur de données électronique est également configuré pour appliquer à l'aide du support robotique un couple sur ladite tête de laser, tel que l'angle entre le faisceau de la tête de laser et la surface de la peau soit maintenu perpendiculaire et/ou dans lequel le traiteur de données électronique est également configuré pour appliquer, à travers le support robotique, une force sur ladite tête de laser de manière longitudinale par rapport à ladite trajectoire telle que la tête multi-laser interchangeable puisse être mue librement le long de ladite trajectoire par l'opérateur à l'aide de ladite poignée ou desdites poignées et/ou dans lequel le traiteur de données électronique est également configuré pour appliquer, à travers le support robotique, une force sur ladite tête de laser le long d'une direction transversale par rapport à ladite trajectoire et vers ladite trajectoire, dans lequel ladite direction transversale est perpendiculaire à la surface de la peau, telle que la distance entre la tête de laser et la surface de la peau soit maintenue constante et égale à une valeur prédéterminée moins une profondeur de tatouage prédéterminée.

11. Dispositif pour l'élimination au laser d'un tatouage ou d'une cicatrice selon l'une quelconque des revendications précédentes dans lequel le capteur de force est assemblé de manière à capter l'intensité et la direction de la force appliquée par l'opérateur directement sur la poignée ou les poignées, ou
assemblé de manière à capter l'intensité et la direction de la force appliquée par l'opérateur indirectement sur la poignée ou les poignées en captant l'intensité et la direction de la force appliquée par l'opérateur sur un joystick assemblé sur la poignée ou les poignées.

12. Dispositif pour l'élimination au laser d'un tatouage ou d'une cicatrice selon l'une quelconque des revendications précédentes, dans lequel le traiteur de données électronique est également configuré pour allier les images capturées par procheinfrarouge et par lumière visible.

13. Dispositif pour l'élimination au laser d'un tatouage ou d'une cicatrice selon l'une quelconque des revendications précédentes, comprenant 3 ou plus de télémètres laser arrangés sur, ou à proximité de, la tête de laser pour déterminer la distance entre la tête de laser et la surface de la peau, ou pour déterminer l'angle entre le faisceau de la tête de laser et la surface de la peau ou pour leur combinaison et/ou comprenant 3 ou plus de télémètres laser arrangés sur, ou à proximité de, la tête multi-laser afin de superposer les points de télémètre laser sur les images de la surface de la peau capturées pour déterminer la taille de pixel de l'image de la surface de la peau ou pour aligner l'image de la surface de la peau capturée avec la tête multi-laser interchangeable ou pour leur combinaison.
